# EUROPÄISCHE PATENTSCHRIFT

(11) **EP 1 977 653 B1**
(45) Veröffentlichungstag und Bekanntmachung des Hinweises auf die Patenterteilung: **04.08.2010**
(21) Anmeldenummer: 08003155.2
(22) Anmeldetag: 21.02.2008
(51) Int. Cl.: A23J 1/14, A23L 1/305, A23L 1/29, A61K 47/42, C09H 3/00, A23K 1/14

(54) **Verfahren zur Gewinnung pflanzlicher Proteine und/oder Peptide, danach hergestellte Proteine und/oder Peptide und Verwendung derselben**
Method for gathering vegetable proteins and/or peptides, proteins and/or peptides produced through this method and usage thereof
Procédé de gain de protéines et/ou de peptides végétaux, protéines et/ou peptides ainsi fabriqués et leur utilisation

(30) Priorität: 15.03.2007 DE 102007012439
(43) Veröffentlichungstag der Anmeldung: 08.10.2008
(73) Patentinhaber: Emsland-Stärke GmbH, 49824 Emlichheim (DE)
(72) Erfinder: Lotz, Martin, Dr., 49824 Emlichheim (DE); Eggengoor, Gerold, 49849 Wilsum (DE)
(74) Vertreter: Scholz, Volker

(56) Entgegenhaltungen:
- EP-A- 1 400 537
- WO-A-01/62101
- WO-A-02/083715
- DE-A1- 2 542 155
- GB-A- 1 580 051
- MENZEL ET AL.: "Abtrennung wertvoller Kartoffelproteine mit Hilfe von Membranadsorbern" BIOSPEKTRUM, Bd. 11, Nr. 1, 2005, Seiten 105-106, XP008095716 DE
- BORUCH ET AL.: "Rückgewinnung der Stickstoffverbindungen aus Kartoffelfruchtwasser" NAHRUNG, Bd. 33, Nr. 1, 1989, Seiten 67-76, XP008095715

## Beschreibung

Die vorliegende Erfindung betrifft ein Verfahren zur Gewinnung pflanzlicher Proteine und/oder Peptide, danach hergestellte Proteine, Peptide und Mischungen derselben sowie Verwendungen derselben.

Für den menschlichen Verzehr sind sowohl tierische als auch pflanzliche Proteine bekannt. Tierische Proteine, wie beispielsweise Hühnerproteine, Milchproteine, wie Casein oder Molke, können jedoch Probleme hinsichtlich BSE, Vogelgruppe und anderer Krankheiten aufweisen. Auch stehen tierische Proteine häufig mit der Auslösung von Allergien im Zusammenhang, auch wenn diese, wie bei einer Lactoseintoleranz, nicht selbst auf dem Protein basieren.

Pflanzliche Proteine haben Probleme mit gentechnisch modifizierten Organismen (GMO), dem Nährwert und ebenfalls mit der Auslösung von Allergien. Das bekannteste pflanzliche Protein ist Sojaprotein. Bei pflanzlichen Proteinen stellt sich ferner häufig das Problem des Geschmacks, beispielsweise bei Soja, so daß die Verwendbarkeit in Lebensmitteln stark eingeschränkt ist. Auch der Einsatz anderer Pflanzenproteine, wie beispielsweise aus Raps, Lupinen oder Kartoffeln, hat sich bislang nicht durchgesetzt. Bei Raps und Leguminosen könnte dies darin begründet sein, daß vor allem der Fettgehalt diese Ausgangsmaterialien zu Ranzigkeit führt.

Chemisch gesehen besteht der Proteinanteil handelsüblicher Produkte, die noch viele andere erwünschte und unerwünschte Stoffe enthalten, aus vielen einzelnen Protein- und Peptidmolekülen, die man zunächst grob phänomenologisch in Globuline und Albumine unterteilen kann. Globuline weisen eine sphärische Gestalt auf, sind daher recht kompakt und in Wasser unlöslich oder zumindest schwer löslich. Albumine weisen eine offene, unregelmäßigere Gestalt auf und sind daher in Wasser löslich. Allgemein faßt man unter Albuminen die löslichen Proteine zusammen. Daneben besteht handelsübliches Eiweiß natürlich aus Protein- und Peptidmolekülen, die ein unterschiedliches Molekulargewicht aufweisen. Dies macht ihre Handhabung, z.B. lebensmitteltechnologisch, recht kompliziert, und eine gesundheitliche Beurteilung kann nur auf Basis des Aminosäurespektrums vorgenommen werden.

Die handelsüblichen Proteine haben somit gemein, daß sie aus einem Gemisch verschiedener Protein- und Peptidmoleküle bestehen und darüber hinaus proteinfremde Bestandteile enthalten, die aus dem ursprünglichen pflanzlichen Ausgangsmaterial stammen. Hierzu gehören z.B. Glucoside, Toxine (Glycoalkaloide, Trypsininhibitoren, usw.), antinutritive Stoffe, wie etwa Phytinsäure, die der menschlichen und tierischen Verdauung Kalzium- und Eisenmineralien entzieht, da diese ausgeschieden und im Darmtrakt nicht resorbiert werden. Ebenfalls enthalten sind Fette und Öle, die teilweise zu Lipoproteinen chemisch verbunden sind, sowie Mineralstoffe.

Bislang gibt es wenige reine Proteine oder Peptide, beispielsweise für eine gesunde Ernährung oder als frei verkäufliche Arzneimittel, die preiswert genug sind, um eine breitere Anwendung zu finden. Der Grund liegt insbesondere darin, daß teure, aus der pharmazeutischen Industrie stammende Aufarbeitungsverfahren eingesetzt werden. Ein weiterer Grund für den hohen Preis sowie eine extrem eingeschränkte Verfügbarkeit ist die Herkunft der Proteinmoleküle, die aus Säugetieren oder aus Menschensekreten, z.B. Blutserum, gewonnen werden, die allesamt die gesuchten Wirkungsproteine in geringer Konzentration enthalten und selbst auch nur in beschränkter Menge zur Verfügung stehen.

In den US 2003113829, US 2003092151, US 2003092152, US 2003092150 und US 2003077265 wird erstmalig beschrieben, wie aus einem Proteingemisch eines pflanzlichen Rohstoffs, hier der Kartoffel, einzelne Proteingruppen relativ rein isoliert werden können.

Beschrieben werden hier die Rohstoffauswahl; die Methode zur Eliminierung von Kunitz- und Bowman-Birk- sowie Carboxypeptidase-Inhibitoren aus Kartoffelprotein durch Erhitzen, Abkühlen, Zentrifugieren und Filtrieren von Kartoffelsaft; Erweiterung der Methode durch Einsatz eines sauren Extraktionsmittels verbunden mit einer Pulverisierung des Pflanzenmaterials in der Extraktionslösung, so daß man Protease-Inhibitor II erhält; Methode zur Steuerung von Ausbeute und Reinheit von Protease-Inhibitor II während der Extraktion durch Auslaugen von Kartoffelschnitzel, Erhitzen der Extraktionslösung, Überwachung von Temperatur, Zeit und Salzkonzentration, Zentrifugation und Membranfiltration; Isolierung und Reinigung von Protease-Inhibitoren II in einer Verfahrensvariante.

Nachteilig bei den bekannten Verfahren ist jedoch, daß zwar einzelne Proteine oder zumindest eng umfaßte Proteingruppen bereitgestellt werden, diese Verfahren jedoch extrem aufwendig, umständlich und teuer sind. Als Nachteil ist auch eine spezielle Auswahl der Kartoffeln anzusehen. Dadurch wird nicht nur die Verfügbarkeit des Rohstoffs eingeschränkt, sondern durch eine erforderliche analytische Kontrolle ist ein zusätzlicher, aufwendiger und teurer Zwischenschritt notwendig. Ferner ist die Logistik aufwendig, da die Kartoffeln frisch verarbeitet werden müssen und nicht gelagert werden können. Auch können die Proteine in den bekannten Verfahren geschädigt werden, da große Mengen an Wärmeenergie benötigt werden, da in verdünnter Extraktionslösung gearbeitet wird und eine hohe Temperatur für eine lange Zeitdauer gehalten werden muß, was zusätzlich große Behälter erforderlich macht. Ebenfalls wird in einem späteren Schritt zusätzliche Energie benötigt, da die zu verarbeitende Stoffmenge auf etwa Raumtemperatur abgekühlt werden muß für die weiteren Verfahrensschritte. Für die Extraktion werden organische Säuren benötigt, die im Abwasser die Umwelt belasten. Ferner muß das Pflanzenmaterial aufwendig auf etwa 100 µm bis 1.500 µm Teilchen zerkleinert werden. Nach der Extraktion und zusätzlich nach der Erhitzung sind Schritte zur Feststoffabtrennung notwendig, um koaguliertes bzw. unlösliches Pflanzenmaterial von der Proteinlösung abzutrennen und die letzte Isolierungsstufe der Ultrafiltration durchführen zu können. Die bekannten Verfahren liefern letztlich nur sehr wenige Proteine, vor allem solche, die nach einer Hitzeeinwirkung von 100°C über eine Zeit von etwa 3 Stunden noch nicht denaturiert sind. Schließlich werden hauptsächlich nur solche Proteine aus dem bekannten Verfahren gewonnen, die die oben erwähnten Kriterien erfüllen, d.h. Protease-Inhibitor II und Carboxypeptidase-Inhibitoren.

Der Erfindung liegt die Aufgabe zugrunde, ein Verfahren zur Gewinnung pflanzlicher Proteine und/oder Peptide bereitzustellen, mit dem die Nachteile aus dem Stand der Technik überwunden werden können. Ebenfalls soll ein Verfahren bereitgestellt werden, mit dem die Gewinnung pflanzlicher Proteine und/oder Peptide auf einer breiteren Rohstoffbasis möglich ist, d.h. nicht nur zur Gewinnung aus Kartoffeln einsetzbar ist, sondern für proteinhaltige Pflanzen im allgemeinen. Insbesondere soll das Verfahren in einer umweltschonenden, energiesparenden, einfachen und preiswerten Weise durchführbar sein und dabei beliebige Proteine und Peptide, rein oder in Mischungen, ohne verfahrensbedingte Einschränkungen gewinnen.

Weitere Aufgaben bestehen in der Bereitstellung von nach dem Verfahren hergestellten Proteinen und/oder Peptiden sowie in der Angabe von Verwendungsmöglichkeiten.

Diese Aufgaben werden durch ein Verfahren nach Anspruch 1, Proteine, Peptide oder Mischungen derselben nach Anspruch 19 sowie Verwendungsmöglichkeiten nach Anspruch 20 gelöst. Bevorzugte Ausführungsformen ergeben sich aus den jeweiligen Unteransprüchen.

Überraschenderweise wurde festgestellt, daß das erfindungsgemäße Verfahren im Gegensatz zu den Verfahren aus dem Stand der Technik vollkommen ohne eine zusätzliche Pflanzenzerkleinerung, Erhitzungs- und Abkühlschritte sowie eine Extraktion mit organischen Zuschlagsstoffen auskommt. Insbesondere wird die Auswahl der Proteine und Peptide, die gewonnen werden sollen, nicht eingeschränkt. Die gezielte Auswahl bestimmter Proteine und/oder Peptide kann durch die Steuerung des Verfahrens zur Selektion durch genaue Einstellung von Verfahrensparametern erreicht werden. Als Ergebnis des Verfahrens werden entweder reine Proteine ohne Anteil fremder Proteine oder beliebig umfangreiche Gemische sich beim Adsorptionsprozeß ähnlich verhaltender Proteine erhalten. Die Reinheit der Proteine kann daher erfindungsgemäß beliebig durch den Desorptionsschritt, z.B. in Form einer Dialyse, eingestellt werden. Dies kann insbesondere dann von Vorteil sein, wenn für medizinische Anwendungen die Qualität eines Vorprodukts ausreichend ist und nur die Endkonfektionierung unter sterilen GMP-Bedingungen erfolgen muß, die der Verfahrensbetreiber ggf. nicht erfüllen kann oder will.

Mit anderen Worten kann mit dem erfindungsgemäßen Verfahren die Fraktionierung der Proteine und/oder Peptide des pflanzlichen Ausgangsmaterials in einzelne Proteine oder Peptide bzw. kleine Gruppen ähnlicher Proteine mit äußerst schonenden Verfahrensschritten erzielt werden, wobei noch stets eine große Vielzahl an Produkten geliefert werden kann und keine teuren oder komplizierten Verfahrensschritte notwendig sind.

Als besonders vorteilhaft hat sich erwiesen, daß erfindungsgemäß eine Fixierung der Ionenaustauschergruppen auf einer Membran anstelle von auf Polymerkügelchen erfolgt. Die Verwendung von lonenaustauschermembranen führt zu einer hohen Durchflußrate, keinem bis geringem Fouling, zu einer äußerst schnellen Beladung, da keine Diffusion notwendig ist, einem geringeren Chemikalienverbrauch für Pufferlösung und Eluenten, eine einfache Handhabung sowie ein einfaches Up-Scaling und die Möglichkeit, Anionen- und Kationenaustauscher zusammenzuschalten, da diese erfindungsgemäß an verschiedene Membrane angebunden sind.

Insbesondere ist es mit dem erfindungsgemäßen Verfahren möglich, nur eine Ionenaustauschermembran einzusetzen, die eine Kationen- oder Anionenaustauschermembran sein kann. Selbstverständlich können auch Kombinationen aus Anionen- und Kationenaustauschermembranen eingesetzt werden. Diese können jeweils in beliebiger Kombination stark oder schwach sauer bzw. alkalisch sein. Vorstellbar ist, daß mehrere Kationenaustauschermembranen und/oder mehrere Anionenaustauschermembranen nacheinander bzw. parallel geschaltet werden. Vorstellbar ist ebenfalls eine Serienschaltung aller Kationenaustauschermembranen und aller Anionenaustauschermembranen, wobei diese beiden Gruppen dann jedoch parallel geschaltet sind. Auch das umgekehrte Vorgehen ist denkbar, wobei Kationenaustauschermembranen und Anionenaustauschermembranen in ihrer jeweiligen Gruppe parallel geschaltet sind, die beiden Gruppen dann jedoch in Serie geschaltet werden. Somit sind alle denkbaren Variationen erfindungsgemäß möglich und im Umfang der Erfindung eingeschlossen.

Weitere Vorteile der Anbindung der lonenaustauschergruppen an eine Membran sind wie folgt:
- Eine hohe Ladungsdichte erlaubt die Verpackung in kleine Volumina, die geringere Kosten bedeuten als z.B. bei der Fixierung auf porösen Polymerkügelchen, die sich in einem Behälter auf einer Siebplatte befinden und so umströmt werden.
- Es ist keine Kapillardiffusion und keine Fick'sche Diffusion notwendig, damit die zu adsorbierenden Moleküle zu den Ionenaustauschermolekülen gelangen, wie z.B. bei porösen Polymerkügelchen aus synthetischen oder natürlichen Polymeren. Es findet lediglich eine Konvektion statt, da die Membrane mit den Ladungsträgern direkt von der Aufgabelösung überströmt wird. Dadurch ist das Adsorptionsverfahren deutlich schneller. Im Umlaufbetrieb ist ein mehrfaches Passieren der Membrane und Poren mit den Ladungsträgern möglich, was das Adsorptionsverfahren und ebenfalls das Desorptionsverfahren deutlich beschleunigt. Die Adsorbermembrane sind wieder- bzw. mehrfach verwendbar und leicht zu reinigen.
- Die Porenweite der Membran kann beliebig zwischen normaler Filtration, Mikro-, Ultra- und Nanofiltration eingestellt werden, je nach Eigenschaften des Fluids und der Stoffe, die behandelt und adsorbiert werden sollen. Daher ist keine Verblockung oder Verstopfung wie bei den Poren von Polymerkügelchen von Ionenaustauschharzen möglich.
- Es gibt unterschiedlichste synthetische Membranmaterialien in fast beliebiger Auswahl, die weitreichende Verfahrensparameterkombinationen aus Druck (transmembran), Temperatur und pH-Wert erlauben.
- Die Bauart der Module, zu denen die Membranen konfektioniert werden und die die technische Bauart der Membranverfahren bestimmen, ist an das Verfahren anpaßbar: Platten-, Querstrom- oder Wickelmodule. Die Auswahl kann unter anderem bezüglich der Viskosität des auf den Membranen zurückbleibenden Feststoffs erfolgen. Ist diese gering, verwendet man bevorzugt ein Modul der Wickelbauart, bei der in einem kleinen Volumen eine große Membranfläche eingebaut werden kann und daher die preiswerteste Modulbauart ist.
- Der Betrieb der Module ist im Chargen- oder Umlaufbetrieb möglich. Im ersten Fall kann nur soviel Aufgabelösung nachgepumpt werden, wie als Permeat aus der Anlage austritt. Erlöscht der Permeatfluß, muß das Retentat von der Membrane entfernt werden, z.B. durch Spülung. Im Umlaufbetrieb läuft die Aufgabelösung zwischen dem Modul und einem Vorlagebehälter im Kreis, so daß die Aufgabelösung mehrmals über die Membrane gegeben werden kann. Der im Retentat enthaltende Feststoff wird aus dem Vorlagebehälter kontinuierlich abgezogen, so daß in den Modulen ein stationärer Zustand zwischen zwei Reinigungszyklen herrscht.

Im folgenden werden die einzelnen Verfahrensschritte des erfindungsgemäßen Verfahrens in bezug auf eine bevorzugte Ausführungsform beschrieben, ohne jedoch den Gegenstand der vorliegenden Anmeldung hierauf beschränken zu wollen.

Das erfindungsgemäße Verfahren zur Gewinnung pflanzlicher Proteine und/oder Peptide wird anhand von Kartoffeln als pflanzliches Ausgangsmaterial beschrieben. Von den rund 2.000 Kartoffelsorten weltweit sind etwa 50 Sorten für die Stärkegewinnung geeignet, da sie überproportional viel Stärke enthalten, in der Regel 17 bis 22 Gew.-%. Grundsätzlich ist aber jede Kartoffelsorte zur Protein- und Peptidgewinnung gemäß dem erfindungsgemäßen Verfahren geeignet.

Nach der Reinigung der Kartoffeln ist der erste Verfahrensschritt zur Stärkegewinnung, daß die Kartoffeln zu einem feinen Brei zerrieben werden. Aus diesem Brei wird als nächstes der Kartoffelsaft (Kartoffelfruchtwasser), der das Protein und/oder Peptid enthält, von den Feststoffen, Stärke und Fasern, abgetrennt. Die Abtrennung der Stärke und Fasern kann beispielsweise durch Zentrifugation oder in Hydrozyklonen erfolgen. Der erhaltene Kartoffelsaft enthält etwa 20 g/L Kartoffelproteine, wovon etwa 40% Patatin, ein majores Speicherprotein, das zu den Glycoproteinen gehört, etwa 50% Protease-Inhibitoren (PI) sowie 10% hochmolekulare Proteine sind, zu denen die Polyphenoloxidasen, Kinasen und Phosphorylasen zählen. Das Patatin hat ein Molekulargewicht von 40 bis 44 kDa und besteht aus 363 Aminosäuren. Bei einem pH-Wert von 7 bis 9 bildet es ein Dimer mit einer Größe von 80 bis 88 kDa. Die PI sind eine heterogene Klasse mit sieben Unterklassen verschiedener Proteine. Ihre Funktion in der Kartoffel ist der Proteinabbau, und somit spielen sie in der Abwehr der Knolle gegen mikrobielle Schädlinge und Insekten eine zentrale Rolle. Die Hinderung des Proteinabbaus wurde im Tiermodell als Wachstumshemmung beobachtet, eine antikarzinogene Wirkung wird diskutiert und das Fördern des Sättigungsgefühls des PI II wird teilweise kommerziell beworben. Die Hauptklassen der PI sind PI i, PI II, Potato-Cystein-PI (PCPI), Kunitz-PI (PKPI), Carboxypeptidase (PCI), Serin-PI (OSPI) und Potato-Aspartyl-PI (PAPI).

Danach wird ein Teil der Proteine und/oder Peptide durch Denaturierung/Koagulation abgetrennt, welcher Teil für eine genauere Auftrennung nicht gewünscht oder benötigt wird. Eine Denaturierung/Koagulation kann beispielsweise durch Verschiebung des pH-Werts, organische Lösungsmittel oder durch Aussalzen erfolgen.

Der erhaltene Kartoffelsaft wird anschließend in einer Mikrofiltrationsmembranvorrichtung geklärt. Dabei ist die Porenweite der Membrane frei wählbar und kann an die gewünschten Produkte, die gewonnen werden sollen, angepaßt werden. Eine Klärung des erhaltenen Kartoffelsafts ist beispielsweise auch durch Zentrifugen jeglicher Bauart möglich, sofern ein klares Zentrifugat mit ausschließlich gelösten Bestandteilen erhalten wird. Diese und alle folgenden Schritte, mit Ausnahme der Trocknung in Schritt g), erfolgen bei einer Temperatur unterhalb der Koagulationstemperatur oder Denaturierungstemperatur der Proteine und/oder Peptide, bevorzugt bei einer Temperatur unter 30°C.

Wesentlicher Kern des erfindungsgemäßen Verfahrens ist die Isolation der Proteine und/oder Peptide aus der wäßrigen Matrix, hier dem Kartoffelsaft, durch Adsorption auf zumindest einer Ionenaustauschermembran, die aus einem synthetischen Polymer aufgebaut ist. Beispiele solcher Membranen sind unter dem Namen Sartobind^{®} von der Firma Sartorius im Handel erhältlich.

Erfindungsgemäß ist es möglich, daß in Schritt c) lediglich ein Teil der Proteine und/oder Peptide aus der wäßrigen Matrix durch Adsorption isoliert werden. Dies hängt stark von den eingesetzten Kationen- bzw. Anionenaustauschermembranen ab.

Ähnlich bedeutend ist ebenfalls die gezielte Desorption der an der Ionenaustauschermembran angebundenen Proteine und/oder Peptide durch speziell angepaßte Eluenten, nachdem zuvor optional Reste des Kartoffelsaftes von der Membran abgewaschen wurden.

Zur Fixierung anionischer Proteine können Ionenaustauschermembrane mit kationischen Gruppen, beispielsweise mit Trimethylgruppen, eingesetzt werden, während für kationische Proteine anionische Gruppen, wie Sulphomethylgruppen, in der Ionenaustauschermembran vorliegen sollten.

Zum mechanischen Schutz der Adsorbermembrane, jedoch auch um deren Standzeit zu verlängern, empfiehlt sich als vorgeschalteter Schritt eine Abtrennung von Feststoffen und suspendierten oder dispergierten Teilchen, wie oben erwähnt. Dies kann durch eine Zentrifuge erfolgen oder durch eine Filtration, letztere in gewöhnlichen Porengrößen von bis zu einer Mikrofiltration. Der Einsatz einer Mikrofiltration geeigneter Porengröße von 0,2 µm hat den Vorteil, sämtliche Protein passieren zu lassen, gleichzeitig aber in der proteinhaltigen Lösung ebenfalls enthaltene Mikroorganismen zu entfernen und somit das Medium keimfrei zu machen. Danach werden die Proteine und/oder Peptide auf den Membranen adsorbiert, indem das Filtrat, Permeat oder geklärte Proteinlösung in die Membranadsorbermodule gepumpt wird. Hierbei sind viele Verfahrensvarianten möglich. Zunächst können die Kationen- und Anionenaustauschermembranmodule parallel oder in Serie geschaltet werden. Die Adsorbermembrane können in Platten-, Querstrom- oder Wickelmodulbauweise konfektioniert sein. Die Zuführung der proteinhaltigen Aufgabelösung kann im Dead-End-Verfahren oder im Umlaufverfahren erfolgen. Ersteres ist zwangsläufig ein Chargenverfahren, letztes kann sowohl chargenweise als auch kontinuierlich erfolgen. Die Porenweite der Adsorbermembrane kann frei gewählt werden, jedoch ist es empfehlenswert, daß sie nicht kleiner ist als die Poren der Vorfiltration, da man sonst Gefahr läuft, auf den Adsorbern Material in Form eines Retentats anzusammeln, das anschließend im Waschschritt zusätzlich entfernt werden muß, und, da es aus potentiellem Produkt besteht, auch Ausbeuteverluste bedeutet. Sind die Adsorbermembrane komplett mit Proteinmolekülen beladen, was analytisch einfach beispielsweise durch Messung der Leitfähigkeit im Auslauf aus den Membranen bzw. beim Dead-End-Betrieb im Permeat festgestellt werden kann, wird der Zulauf der Aufgabelösung unterbrochen, und die Membrane kann optional gewaschen werden, um Verunreinigungen zu entfernen. Das Waschen kann durch Wasser oder eine Reinigungslösung erfolgen, jedoch sollte diese die Proteine nicht denaturieren.

Die an den Membranen anhaftenden Produkte können dann wie bei einem gewöhnlichen Chromatographieverfahren nach einander und selektiv mit mehreren Eluenten desorbiert werden. Dies geschieht bevorzugt mit Salzlösung, deren Zusammensetzung und Konzentration von den zu eluierenden Proteinen und Peptiden abhängt. Typischerweise werden Natriumchlorid- und Ammoniumchloridlösungen verwendet, jedoch ist hier die Auswahl nahezu unbegrenzt und durch die Eigenschaften der Proteine vorgegeben. Auch die Zugabe von Puffersalzen bzw. Pufferlösungen, z.B. Phosphatpuffer, ist möglich. Damit die eluierten Proteine nicht denaturieren, sollten sie im Eluenten nur in geringer Konzentration vorliegen. Daher ist vor dem Trocknen ein Konzentrierungsschritt vorteilhaft. Darüberhinaus kann durch Waschen mit destilliertem Wasser oder Leitungswasser die Reinheit der so isolierten Proteine beliebig eingestellt werden. Nimmt man für diese beiden Verfahrensschritte eine Ultrafiltrationsmembrane in einer Platten-, Querstrom- oder Wickelmodulbauweise in Umlaufbetrieb, können hier Filtration und Konzentration gleichzeitig durchgeführt werden, zum Beispiel indem ständig maximal soviel Waschwasser in die Vorlage nachgefüllt wird, wie Permeat durch die Poren der Ultrafiltrationsmembrane durchtritt. Die Reinheitskontrolle kann effektiv durch Messung der elektrischen Leitfähigkeit im Permeat kontrolliert werden.

Im nächsten Verfahrensschritt erfolgt eine Isolierung des Produkts aus den Eluenten, zum Beispiel durch Trocknung oder durch Trennung von Eluent und Proteinmolekülen auf einer Membran mit geeigneter Porenweite, die bevorzugt im Bereich von Ultra- oder Nanofiltration bis zu Umkehrosmose reicht, diafiltriert und konzentriert oder nur konzentriert oder nur diafiltriert werden.

Als letzter Schritt schließt sich optional eine Trocknung an, wobei vorteilhaft eine schonende Gefriertrocknung oder eine Sprühtrocknung eingesetzt werden kann. Andere Trocknungsarten sind ebenfalls möglich, jedoch sollte eine intensive Wärmeeinwirkung vermieden werden, die zu einer Produktschädigung führen könnte.

Die folgenden Beispiele veranschaulichen weiter das erfindungsgemäße Verfahren in größerem Detail.

### Beispiel 1

Ein Anionentauschermodul mit 80 m² Fläche kann bei einer Bindekapazität von 0,4 mg Protein/cm² 320 g Protein binden. 50% der Proteine in Kartoffelsaft sind PI, das sind etwa 10 g/l. Nach Aufgabe von 32 1 Kartoffelsaft ist danach die Kapazität erschöpft. Dies dauert bei einer typischen Durchflußrate von 300 1/h etwa 6,5 Minuten. Abschließend können die PI-Proteine eluiert werden.

### Beispiel 2

Ein Kationentauschermodul mit 80 m² Fläche kann bei einer Bindekapazität von 0,25 mg Protein/cm² 200 g Protein binden. 40% der Proteine in Kartoffelsaft sind Patatin, das sind etwa 8 g/l. 3,3 m² Membran sind für die vollständige Bindung des Patatins aus 1 1 Kartoffelsaft notwendig. Auf 330 m² kann daher 1 kg Patatin aus 125 1 Saft adsorbiert werden. Danach kann das Patatin eluiert werden.

### Beispiel 3

Ein großer Vorteil des erfindungsgemäßen Verfahrens ist die Mehrfachverwendung sowohl der Membranadsorber als auch der Waschlösung und der Eluenten.

Ein Kationenaustauschermodul mit 15 cm² Fläche wird mit 1,5 ml einer BSA-Lösung (BSA = Rinderserumalbumin) mit einer Konzentration von 10 mg/ml beladen. Dies liegt etwas unterhalb der maximalen Beladung von etwa 20 mg. Das Schema zur Identifizierung der Langzeitstabilität wird durchgeführt, indem beladen, gespült, eluiert und gespült wird. Spülflüssigkeit ist ein 50 mM Kaliumphosphatpuffer bei pH 7, Eluent ist eine 1M NaCl-Lösung im gleichen Puffer. Ein Zyklus dauert 21,5 Minuten. Mit der Zeit verbreitern sich naturgemäß die Eluierungspeaks, und bis zu 65 Zyklen sind möglich, ohne die Membran zu verblocken, und ohne daß ein Durchbruch erfolgte. Verlängert man den Spülschritt um 5 Minuten sind über 100 Zyklen ohne Membranreinigung möglich. Eine Verblockung tritt ab dem 108. Zyklus ein. Nach einer Reinigung mit 0,5 M Natronlauge war die Membran wieder frei, so daß wieder mit dem Produktionsverfahren begonnen werden konnte. So sind mehrere Tausend Zyklen mit einem Adsorbermodul möglich, bevor es verschlissen ist.

### Beispiel 4

Ein Nachteil ist der hohe Wasser- und Salzverbrauch beim Spülen und Eluieren. Daher ist die Mehrfachverwendung der Lösungen sehr vorteilhaft. Das Eluat nach einem Zyklus und einer Beladung mit 0,4 mg Protein/ml wird erneut zur Eluierung eingesetzt, und die Beladung betrug nun 0,86 mg/ml. Nach der vierten Eluierung stieg die Konzentration auf 1,2 mg/ml an. Die Einsparung des Eluenten (Wasser, Salz und Puffer) beträgt somit 75%.

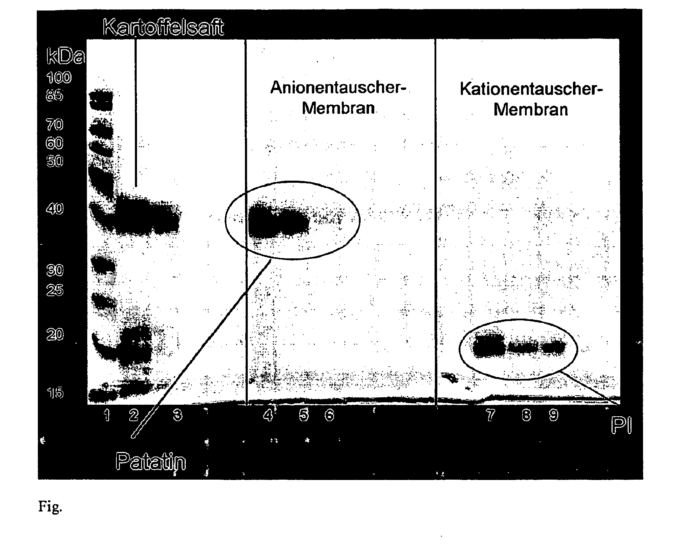

Die in der Anlage beigefügte Figur 1 zeigt ein SDS-PAGE auf Gelbasis mit der Darstellung der gesamten Proteine in Kartoffelsaft vor erfindungsgemäßer Verarbeitung sowie der auf den Kationen- und Anionenaustauscheradsorbermembranen fixierten und wieder eluierten Proteinen und Proteinfraktionen, die gemäß dem erfindungsgemäßen Verfahren erhalten werden.

Wie aus der Figur 1 zu entnehmen ist, konnten über die Anionenauschermembran gezielt Patatin und über die Kationentauschermembran gezielt PI isoliert und im wesentlichen rein abgetrennt werden, was die Vorteile des erfindungsgemäßen Verfahrens nochmals eindrucksvoll unterstreicht.

Die erfindungsgemäß gewonnenen Proteine und/oder Peptide können beispielsweise in funktionellen Nahrungsmitteln eingesetzt werden, das heißt Nahrungsmitteln mit positiver physiologischer Wirkung. Sie können auch zur Krankheitsbekämpfung und -prävention sowie zur Steigerung der Leistung und des Wohlbefindens verwendet werden. Eine bevorzugte Verwendung der erfindungsgemäß hergestellten Proteine und/Peptide könnte in einer Arzneimittelform sein, beispielsweise in Kapselform. Hier ist insbesondere der Protease-Inhibitor II von Interesse, da seine appetitzügelnde Wirkung bekannt ist und gut in beispielsweise eine Hartgelkapsel eingebracht werden kann.

Die in der vorstehenden Beschreibung, in den Ansprüchen sowie in der Zeichnung offenbarten Merkmale der Erfindung können sowohl einzeln als auch in jeder beliebigen Kombination für die Verwirklichung der Erfindung in ihren verschiedenen Ausführungsformen wesentlich sein.

## Patentansprüche

1. Verfahren zur Gewinnung pflanzlicher Proteine und/oder Peptide, welches die Schritte umfaßt:
a) Vorlegen eines Proteine und/oder Peptide enthaltenden, pflanzlichen Ausgangsmaterials in einer wässrigen Matrix;
b) optional Abtrennen von festen Bestandteilen aus der wässrigen Matrix und/oder Klären der wässrigen Matrix;
c) Isolieren von zumindest einem Teil der Proteine und/oder Peptide aus der wässrigen Matrix durch Adsorption auf zumindest einer Ionenaustauschermembran aus synthetischem Polymer;
d) optional Waschen der Ionenaustauschermembran, um Verunreinigungen zu entfernen;
e) Desorbieren der Proteine und/oder Peptide von der Ionenaustauschermembran mit zumindest einem Eluenten;
f) Isolieren der Proteine und/oder Peptide vom Eluenten; und
g) optional Trocknen der isolierten Proteine und/oder Peptide,
wobei die Desorption einzelner Proteine und/oder Peptide oder Gruppen von diesen in Schritt e) nacheinander und selektiv unter Verwendung mehrerer unterschiedlicher Eluenten erfolgt und vor Schritt c) ein Teil der Proteine und/oder Peptide aus der wässrigen Matrix durch Denaturierung/Koagulation ausgefällt und abgetrennt wird.

2. Verfahren nach Anspruch 1, **dadurch gekennzeichnet, daß** die wässrige Matrix durch Zerreiben des pflanzlichen Ausgangsmaterials zu einem Brei oder Vermahlen von, insbesondere trockenen, Ausgangsmaterialien zu einem Mehl und Quellen in Wasser sowie Abtrennen von festen Bestandteilen erhalten wird.

3. Verfahren nach Anspruch 2, **dadurch gekennzeichnet, daß** die festen Bestandteile Stärke und Fasern aus dem pflanzlichen Ausgangsmaterial sind.

4. Verfahren nach einem der vorangehenden Ansprüche, **dadurch gekennzeichnet, daß** das pflanzliche Ausgangsmaterial ausgewählt wird aus proteinhaltigen Pflanzen, bevorzugt Kartoffeln, Leguminosen, Soja, Raps und Mischungen derselben, besonders bevorzugt Kartoffeln.

5. Verfahren nach Anspruch 4, **dadurch gekennzeichnet, daß** Leguminosen ausgewählt werden aus Erbsen, Bohnen, Lupinen, Soja und Mischungen derselben.

6. Verfahren nach einem der vorangehenden Ansprüche, **dadurch gekennzeichnet, daß** das Klären in Schritt b) in einer Mikrofiltrationsmembranvorrichtung erfolgt.

7. Verfahren nach einem der vorangehenden Ansprüche, **dadurch gekennzeichnet, daß** zumindest die Schritt a)-f) bei einer Temperatur unterhalb der Koagulations- oder Denaturierungstemperatur der Proteine und/oder Peptide, bevorzugt bei einer Temperatur von unter 30°C durchgeführt werden.

8. Verfahren nach einem der vorangehenden Ansprüche, **dadurch gekennzeichnet, daß** der Schritt c) und/oder e) in einem Chargen- oder Umlaufverfahren betrieben wird bzw. werden.

9. Verfahren nach einem der vorangehenden Ansprüche, **dadurch gekennzeichnet daß** in Schritt c) zumindest eine Kationenaustauscher- und zumindest eine Anionenaustauschermembran eingesetzt werden.

10. Verfahren nach Anspruch 9, **dadurch gekennzeichnet, daß** die Kationenaustauschermembran und die Anionenaustauschermembran parallel oder in Serie betrieben werden.

11. Verfahren nach einem der vorangehenden Ansprüche, **dadurch gekennzeichnet, daß** jede Ionenaustauschermembran in einem Absorbermodul vorliegt, bevorzugt einem Platten-, Querstrom- oder Wickelmodul.

12. Verfahren nach einem der vorangehenden Ansprüche, **dadurch gekennzeichnet, daß** die Porenweite der Ionenaustauschermembran eingestellt wird, um Mikro-, Ultra- oder Nanofiltration zu erreichen.

13. Verfahren nach einem der vorangehenden Ansprüche, **dadurch gekennzeichnet, daß** der Eluent ausgewählt wird aus einer wässrigen Salzlösung, bevorzugt einer Natriumchlorid- und Ammoniumchloridlösung.

14. Verfahren nach einem der vorangehenden Ansprüche, **dadurch gekennzeichnet, daß** das Isolieren in Schritt f) über eine Membranfiltration oder eine Trocknung erfolgt.

15. Verfahren nach einem der vorangehenden Ansprüche, **dadurch gekennzeichnet, daß** das Trocknen in Schritt g) über eine Sprühtrocknung oder Gefriertrocknung erfolgt.

16. Verfahren nach einem der vorangehenden Ansprüche, **dadurch gekennzeichnet, daß** die Denaturierung/Koagulation durch Verschiebung des pH-Wertes, organische Lösungsmittel oder durch Aussalzen erfolgt.

## Claims

1. Method of obtaining vegetable proteins and/or peptides, comprising the steps of:
a) providing a vegetable starting material containing proteins and/or peptides in an aqueous matrix;
b) optionally separating off the solid components from said aqueous matrix and/or clarifying said aqueous matrix;
c) isolating at least part of the proteins and/or peptides from the aqueous matrix by adsorption on at least one ion exchange membrane made of a synthetic polymer;
d) optionally rinsing the ion exchange membrane in order to remove impurities;
e) desorbing the proteins and/or peptides from the ion exchange membrane with at least one eluent;
f) isolating the proteins and/or peptides from the eluent; and
g) optionally drying the isolated proteins and/or peptides,
the desorption of individual proteins and/or peptides or groups thereof taking place in step e) consecutively and selectively, using several different eluents and before step c), some of the proteins and/or peptides are precipitated and separated off from the aqueous matrix by denaturing/coagulation.

2. Method according to claim 1, **characterised in that** that the aqueous matrix is obtained by grinding the vegetable starting material to a pulp or milling starting materials, especially dry ones, into a flour and swelling in water and separating off the solid components.

3. Method according to claim 2, **characterised in that** the solid components comprise starch and fibres from the vegetable starting material.

4. Method according to one of the preceding claims, **characterised in that** the vegetable starting material is selected from protein-containing plants, preferably potatoes, legumes, soya, rapeseed and mixtures thereof, particularly preferably potatoes.

5. Method according to claim 4, **characterised in that** the legumes are selected from peas, beans, lupins, soya and mixtures thereof.

6. Method according to one of the preceding claims, **characterised in that** the clarification in step b) is performed in a microfiltration membrane apparatus.

7. Method according to one of the preceding claims, **characterised in that** at least steps a)-f) are carried out at a temperature below the coagulation or denaturing temperature of the proteins and/or peptides, preferably at a temperature of less than 30°C.

8. Method according to one of the preceding claims, **characterised in that** steps c) and/or e) is/are operated in a batch or circulating process.

9. Method according to one of the preceding claims, **characterised in that** at least one cation exchange membrane and at least one anion exchange membrane are used in step c).

10. Method according to claim 9, **characterised in that** the cation exchange membrane and the anion exchange membrane are operated in parallel or in series.

11. Method according to one of the preceding claims, **characterised in that** each ion exchange membrane is present in an absorber module, preferably a plate, cross-flow or coil module.

12. Method according to one of the preceding claims , **characterised in that** the pore width of the ion exchange membrane is adjusted in order to achieve microfiltration, ultrafiltration or nanofiltration.

13. Method according to one of the preceding claims, **characterised in that** the eluent is selected from an aqueous salt solution, preferably a sodium chloride and ammonium chloride solution.

14. Method according to one of the preceding claims, **characterised in that** the isolation in step f) is performed by membrane filtration or drying.

15. Method according to one of the preceding claims, **characterised in that** the drying in step g) is performed by spray-drying or freeze-drying.

16. Method according to one of the preceding claims, **characterised in that** the denaturing/coagulation is effected by shifting the pH value, organic solvents or by salting out.

## Revendications

1. Procédé d'obtention de protéines et/ou de peptides végétaux, comprenant les étapes suivantes :
a) placement d'un matériau de départ végétal contenant une protéine et/ou un peptide dans une matrice aqueuse ;
b) séparation éventuelle des composants solides de la matrice aqueuse et/ou décantation de la matrice aqueuse ;
c) isolement d'au moins une partie des protéines et/ou peptides de la matrice aqueuse par adsorption sur au moins une membrane échangeuse d'ions en polymère synthétique ;
d) lavage éventuel de la membrane échangeuse d'ions pour éliminer les impuretés ;
e) désorption des protéines et/ou peptides de la membrane échangeuse d'ions avec au moins un éluant ;
f) isolement des protéines et/ou peptides des éluants ; et
g) séchage éventuel des protéines et/ou peptides isolés,
la désorption de protéines et/ou peptides individuels ou de groupes de ceux-ci s'effectuant à l'étape e) de façon consécutive et sélective en utilisant plusieurs éluants différents et avant l'étape c), une partie des protéines et/ou peptides étant précipitée et séparée de la matrice aqueuse par dénaturation/coagulation.

2. Procédé selon la revendication 1, **caractérisé en ce que** la matrice aqueuse est obtenue par trituration du matériau de départ végétal en une pâte ou broyage des matériaux de départ, notamment secs, en une farine et gonflement dans l'eau et séparation des composants solides.

3. Procédé selon la revendication 2, **caractérisé en ce que** les composants solides sont de l'amidon et des fibres du matériau de départ végétal.

4. Procédé selon l'une des revendications précédentes, **caractérisé en ce que** le matériau de départ végétal est choisi parmi des plantes contenant des protéines, en particulier, des pommes de terre, des légumineuses, du soja, du colza et leurs mélanges, de manière particulièrement préférée, des pommes de terre.

5. Procédé selon la revendication 4, **caractérisé en ce que** les légumineuses sont choisies parmi les pois, les haricots, les lupins, le soja et leurs mélanges.

6. Procédé selon l'une des revendications précédentes, **caractérisé en ce que** la décantation de l'étape b) s'effectue dans un dispositif à membrane de microfiltration.

7. Procédé selon l'une des revendications précédentes, **caractérisé en ce que** au moins les étapes a) à f) sont réalisées à une température inférieure à la température de coagulation ou de dénaturation des protéines et/ou des peptides, de préférence à une température inférieure à 30°C.

8. Procédé selon l'une des revendications précédentes, **caractérisé en ce que** l'étape c) et/ou e) est/sont réalisée(s) dans un procédé en lot ou en continu.

9. Procédé selon l'une des revendications précédentes, **caractérisé en ce qu'**à l'étape c), au moins une membrane échangeuse de cation et au moins une membrane échangeuse d'anions est utilisée.

10. Procédé selon la revendication 9, **caractérisé en ce que** la membrane échangeuse de cations et la membrane échangeuse d'anions fonctionnent en parallèle ou en série.

11. Procédé selon l'une des revendications précédentes, **caractérisé en ce que** chaque membrane échangeuse d'ions se présente dans un module absorbant, de préférence dans un module en plaque, à courant transversal ou à enroulement.

12. Procédé selon l'une des revendications précédentes, **caractérisé en ce que** la largeur de pores de la membrane échangeuse d'ions est ajustée de façon à obtenir une micro-, une ultra- ou une nano-filtration.

13. Procédé selon l'une des revendications précédentes, **caractérisé en ce que** l'éluant est choisi parmi les solutions physiologiques aqueuses, de préférence une solution de chlorure de sodium et une solution de chlorure d'ammonium.

14. Procédé selon l'une des revendications précédentes, **caractérisé en ce que** l'isolement de l'étape f) s'effectue par filtration sur membrane ou par séchage.

15. Procédé selon l'une des revendications précédentes, **caractérisé en ce que** le séchage de l'étape g) s'effectue par séchage par pulvérisation ou lyophilisation.

16. Procédé selon l'une des revendications précédentes, **caractérisé en ce que** la dénaturation/coagulation s'effectue par décalage du pH, par un solvant organique ou par relargage.
